# EUROPEAN PATENT APPLICATION

(11) **EP 4 527 837 A1**
(43) Date of publication of application: **26.03.2025**
(21) Application number: 23807052.8
(22) Date of filing: 19.05.2023
(51) Int. Cl.: C07D 405/04, C07D 407/02, C07D 251/40, A61P 9/04, A61P 9/10, A61K 31/53

(54) **CRYSTAL FORMS OF TRIAZINE DIONE DERIVATIVE AND PREPARATION METHOD THEREFOR**

(30) Priority: 20.05.2022 CN 202210553394
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHANG, Baolei, Shanghai 200245 (CN); XI, Zhuoxun, Shanghai 200245 (CN); FENG, Jun, Shanghai 200245 (CN); ZHAO, Miaomiao, Lianyungang, Jiangsu 222047 (CN); DU, Zhenxing, Lianyungang, Jiangsu 222047 (CN)
(74) Representative: Regimbeau
(86) International application number: PCT/CN2023/095172
(87) International publication number: WO 2023/222103

(57) **Abstract**

The present disclosure relates to crystal forms of a triazine dione derivative and a preparation method therefor. Specifically, the present disclosure relates to different crystal forms of (S)-6-((1-(2-fluoro-5-methylphenyl)ethyl)amino)-3-(tetrahydro-2H-pyran-4-yl)-1,3,5-triazine-2,4(1H,3H)-dione and a preparation method therefor. The crystal forms of (S)-6-((1-(2-fluoro-5-methylphenyl)ethyl)amino)-3-(tetrahydro-2H-pyran-4-yl)-1,3,5-triazine-2,4(1H,3H)-dione provided by the present disclosure have good stability and can be better used for clinical treatment.

## Description

### TECHNICAL FIELD

The present disclosure belongs to the field of pharmaceuticals and relates to a crystal form of a triazine dione derivative and specifically to a crystal form of (S)-6-((1-(2-fluoro-5-methylphenyl)ethyl)amino)-3-(tetrahydro-2H-pyran-4-yl)-1,3,5-triazine-2,4(1H,3H)-dione and a preparation method therefor.

### BACKGROUND

Hypertrophic cardiomyopathy (HCM) is a dominant hereditary cardiomyopathy associated with genetic mutations. Its global incidence is about 0.2%. It is the biggest cause of sudden death in young people under 35 (Tuohy, CV. et al., Eur J Heart Fail, 22, 2020, 228-240). Clinically, it is characterized by asymmetric left ventricular wall hypertrophy, typical thickening of ventricular septum, reduced ventricular cavity size, obstructed left ventricular blood filling, and decreased ventricular diastolic compliance. The disease is classified into obstructive and non-obstructive hypertrophic cardiomyopathy according to the presence or absence of obstruction in the outflow tract of the left ventricle. In clinical practice, β-blockers and calcium channel blockers are commonly used to reduce cardiac contraction and alleviate symptoms in the treatment of hypertrophic cardiomyopathy. However, all of these treatments are targeted at the symptoms rather than the root cause. When HCM becomes advanced, the patient has to have a heart transplant (Ramaraj, R. Cardiol Rev, 16, 2008, 172-180). Therefore, it is very urgent to find a treatment targeted at the root cause of HCM.

Research has found that 70% of HCM patients are caused by mutations in the sarcomeric protein genes. Multiple site mutations are found in 5-7% of patients. More than 70 pathogenic mutations have been identified, but most of them are family-specific, and only a few hotspots are identified, e.g., MYH7 R403Q and R453C mutations (Frey, N. et al., Nat Rev Cardiol, 9, 2011, 91-100; Sabater-Molina, M. et al., Clin Genet, 93, 2018, 3-14). A study on the pathogenic probability of genetic mutation reveals that about 30% of patients contain mutations in the MYH7 gene. MYH7 causes early onset of disease and more severe myocardial hypertrophy than other sarcomeric protein genes. Myosin is the constituent of thick filaments in myofibrils and plays an important role in the motion of muscles. The molecule takes the shape of a beansprout and consists of two heavy chains and several light chains. The myosin heads bind actin to form cross-bridges, which greatly increase the ATPase activity of myosin. Myosin catalyzes ATP hydrolysis and the energy produced causes the cross-bridges to slide and thus muscle contraction. Research findings show that MYH7 gene mutations can cause an increase in the activity of myosin ATPase, a decrease in the proportion of the super-relaxed state (SRX) of myosin, and more cross-bridges between myosin and actin, and thus abnormal systolic function (Green, EM. et al., Science, 351, 2016, 617-621; Sommese, RF. et al., Proc Natl Acad Sci U S A, 110, 2013, 12607-12612). Myosin is therefore an important target for the treatment of hypertrophic cardiomyopathy.

Application No. WO2022105852 provides a series of triazine dione derivatives, including, (S)-6-((1-(2-fluoro-5-methylphenyl)ethyl)amino)-3-(tetrahydro-2H-pyran-4-yl)-1,3,5-triazine-2,4(1H,3H)-dione, and they are structurally characterized. Additionally, (S)-6-((1-(2-fluoro-5-methylphenyl)ethyl)amino)-3-(tetrahydro-2H-pyran-4-yl)-1,3,5-triazine-2,4(1H,3H)-dione was also biologically evaluated in the application. The results show that the compound has a very good inhibitory effect on myosin ATPase.

The structure of a crystal form of a pharmaceutical active ingredient generally affects the chemical stability of the drug, and the differences in crystallization conditions and storage conditions may cause changes in the structure of the crystal form of the compound and sometimes generation of other crystal forms. Generally, amorphous drug products have no regular crystal form structure and often have other defects, such as poor product stability, fine powder, difficulty in filtration, ease of agglomeration and poor flowability. Therefore, it is necessary to improve various properties of the above products, and intensive research is needed to find crystal forms with relatively high crystal form purity and good physicochemical stability.

### SUMMARY

The present disclosure provides a crystal form of a myosin inhibitor (S)-6-((1-(2-fluoro-5-methylphenyl)ethyl)amino)-3-(tetrahydro-2H-pyran-4-yl)-1,3,5-triazine-2,4(1H,3H)-dione, as well as a preparation method therefor and use thereof.

The present disclosure provides a crystal form of (S)-6-((1-(2-fluoro-5-methylphenyl)ethyl)amino)-3-(tetrahydro-2H-pyran-4-yl)-1,3,5-triazine-2,4(1H,3H)-dione.

In some embodiments, the present disclosure provides a crystal form A of (S)-6-((1-(2-fluoro-5-methylphenyl)ethyl)amino)-3-(tetrahydro-2H-pyran-4-yl)-1,3,5-triazine-2,4(1H,3H)-dione, and an X-ray powder diffraction pattern represented by 2θ angles, which are diffraction angles, of the crystal form has characteristic peaks at 9.2, 10.9, 18.6, 20.1, 22.0, and 26.9, optionally 9.2, 10.9, 13.3, 16.9, 18.6, 19.2, 20.1, 22.0, 26.9, and 35.6, and optionally 9.2, 10.9, 13.3, 16.9, 18.6, 19.2, 20.1, 20.6, 22.0, 26.9, 28.0, and 35.6.

In some embodiments, the present disclosure provides a crystal form A of (S)-6-((1-(2-fluoro-5-methylphenyl)ethyl)amino)-3-(tetrahydro-2H-pyran-4-yl)-1,3,5-triazine-2,4(1H,3H)-dione, and an X-ray powder diffraction pattern represented by 2θ angles, which are diffraction angles, of the crystal form has characteristic peaks at 9.2, 10.9, 18.5, 20.1, 22.0, and 26.9, optionally 9.2, 10.9, 13.3, 16.8, 18.5, 19.1, 20.1, 22.0, 26.9, and 35.5, and optionally 9.2, 10.9, 13.3, 16.8, 18.5, 19.1, 20.1, 20.6, 22.0, 26.9, 27.9, and 35.5.

In some embodiments, the present disclosure provides a crystal form B of (S)-6-((1-(2-fluoro-5-methylphenyl)ethyl)amino)-3-(tetrahydro-2H-pyran-4-yl)-1,3,5-triazine-2,4(1H,3H)-dione, and an X-ray powder diffraction pattern represented by 2θ angles, which are diffraction angles, of the crystal form has characteristic peaks at 11.1, 15.9, 18.8, 20.4, 22.2, and 26.8, optionally 6.6, 9.5, 11.1, 15.9, 18.8, 20.4, 21.0, 22.2, and 26.8, and optionally 6.6, 9.5, 11.1, 13.3, 14.0, 15.9, 18.8, 19.0, 19.8, 20.4, 21.0, 22.2, 26.8, and 28.0.

In some embodiments, the present disclosure provides a crystal form C of (S)-6-((1-(2-fluoro-5-methylphenyl)ethyl)amino)-3-(tetrahydro-2H-pyran-4-yl)-1,3,5-triazine-2,4(1H,3H)-dione, and an X-ray powder diffraction pattern represented by 2θ angles, which are diffraction angles, of the crystal form has characteristic peaks at 8.3, 14.4, 16.8, 17.8, 19.3, 22.3, and 23.1.

In some embodiments, the present disclosure provides a crystal form D of (S)-6-((1-(2-fluoro-5-methylphenyl)ethyl)amino)-3-(tetrahydro-2H-pyran-4-yl)-1,3,5-triazine-2,4(1H,3H)-dione, and an X-ray powder diffraction pattern represented by 2θ angles, which are diffraction angles, of the crystal form has characteristic peaks at 7.8, 8.7, 9.7, 13.5, 14.0, and 22.9, optionally 7.8, 8.7, 9.7, 13.5, 14.0, 16.7, 19.6, 20.2, and 22.9, and optionally 7.8, 8.7, 9.7, 13.5, 14.0, 15.5, 16.7, 17.5, 19.6, 20.2, 22.9, and 23.9.

In some embodiments, the present disclosure provides a crystal form E of (S)-6-((1-(2-fluoro-5-methylphenyl)ethyl)amino)-3-(tetrahydro-2H-pyran-4-yl)-1,3,5-triazine-2,4(1H,3H)-dione, and an X-ray powder diffraction pattern represented by 2θ angles, which are diffraction angles, of the crystal form has characteristic peaks at 6.9, 9.3, 14.2, 17.1, 18.0, and 25.0, optionally 6.9, 9.3, 13.5, 14.2, 17.1, 18.0, 22.4, 23.2, and 25.0, and optionally 6.9, 9.3, 13.5, 14.2, 16.1, 17.1, 18.0, 21.1, 22.4, 23.2, 23.8, and 25.0.

In an optional embodiment, for the crystal forms of the compound represented by formula (I) provided by the present disclosure, the 2θ angles have a margin of error of ±0.2.

The present disclosure provides a preparation method for the crystal form A of (S)-6-((1-(2-fluoro-5-methylphenyl)ethyl)amino)-3-(tetrahydro-2H-pyran-4-yl)-1,3,5-triazine-2,4(1H,3H)-dione, the method being selected from the group consisting of any one of the following methods:
method 1:
   (a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (1), and dissolving the compound by stirring or heat, the solvent (1) being selected from the group consisting of at least one of 7% water/ethanol, tetrahydrofuran, 10% water/isopropanol, 10% water/acetone, methanol, ethanol, and 10% water/methanol, and
   (b) crystallizing;
or, method 2:
   (a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (2), and dissolving the compound by stirring or heat, the solvent (2) being selected from the group consisting of 10% water/acetone and dichloromethane, and
   (b) adding a solvent (3), and crystallizing, the solvent (3) being selected from the group consisting of at least one of water, methanol, and ethanol;
or, method 3:
   (a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (4), the solvent (4) being selected from the group consisting of at least one of water, methanol, ethanol, isopropanol, acetone, ethyl acetate, acetonitrile, isopropyl acetate, methyl tert-butyl ether, methyl isobutyl ketone, n-heptane, cyclohexane, 1,4-dioxane, 10% water/methanol, and 7% water/ethanol, and
   (b) triturating, and crystallizing.

The present disclosure provides a preparation method for the crystal form B of (S)-6-((1-(2-fluoro-5-methylphenyl)ethyl)amino)-3-(tetrahydro-2H-pyran-4-yl)-1,3,5-triazine-2,4(1H,3H)-dione, the method being selected from the group consisting of any one of the following methods:
method 1:
   (a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (5), and dissolving the compound by stirring or heat, the solvent (5) being selected from the group consisting of ethanol, and
   (b) crystallizing;
or, method 2:
   (a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (6), and dissolving the compound by stirring or heat, the solvent (6) being selected from the group consisting of dichloromethane, and
   (b) adding a solvent (7) and crystallizing, the solvent (7) being selected from the group consisting of at least one of ethyl acetate, acetone, acetonitrile, and methyl isobutyl ketone.

The present disclosure provides a preparation method for the crystal form C of (S)-6-((1-(2-fluoro-5-methylphenyl)ethyl)amino)-3-(tetrahydro-2H-pyran-4-yl)-1,3,5-triazine-2,4(1H,3H)-dione, the method comprising:
(a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (9), and dissolving the compound by stirring or heat, the solvent (9) being selected from the group consisting of dichloromethane, and
(b) adding a solvent (10), and crystallizing, the solvent (10) being selected from the group consisting of isopropyl acetate.

The present disclosure provides a preparation method for the crystal form D of (S)-6-((1-(2-fluoro-5-methylphenyl)ethyl)amino)-3-(tetrahydro-2H-pyran-4-yl)-1,3,5-triazine-2,4(1H,3H)-dione, the method comprising:
(a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (11), the solvent (11) being selected from the group consisting of dichloromethane, and
(b) adding a solvent (12), triturating, and crystallizing, the solvent (12) being selected from the group consisting of methyl tert-butyl ether.

The present disclosure provides a preparation method for the crystal form E of (S)-6-((1-(2-fluoro-5-methylphenyl)ethyl)amino)-3-(tetrahydro-2H-pyran-4-yl)-1,3,5-triazine-2,4(1H,3H)-dione, the method comprising:
(a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent (13), the solvent (13) being selected from the group consisting of dichloromethane, and
(b) adding a solvent (14), triturating, and crystallizing, the solvent (14) being selected from the group consisting of n-heptane.

In certain embodiments, the preparation methods for the crystal forms described in the present disclosure also comprise one or more of a filtration step, a washing step, and a drying step.

The present disclosure also provides a pharmaceutical composition prepared from the aforementioned crystal forms of (S)-6-((1-(2-fluoro-5-methylphenyl)ethyl)amino)-3-(tetrahydro-2H-pyran-4-yl)-1,3,5-triazine-2,4(1H,3H)-dione.

The present disclosure also provides a pharmaceutical composition, the pharmaceutical composition comprising the aforementioned crystal forms of (S)-6-((1-(2-fluoro-5-methylphenyl)ethyl)amino)-3-(tetrahydro-2H-pyran-4-yl)-1,3,5-triazine-2,4(1H,3H)-dione or a mixture thereof, or crystal forms of (S)-6-((1-(2-fluoro-5-methylphenyl)ethyl)amino)-3-(tetrahydro-2H-pyran-4-yl)-1,3,5-triazine-2,4(1H,3H)-dione prepared by the aforementioned methods, and optionally a pharmaceutically acceptable excipient.

The present disclosure also provides a preparation method for a pharmaceutical composition, the method comprising a step of mixing the aforementioned crystal forms of (S)-6-((1-(2-fluoro-5-methylphenyl)ethyl)amino)-3-(tetrahydro-2H-pyran-4-yl)-1,3,5-triazine-2,4(1H,3H)-dione or a mixture thereof, or crystal forms of (S)-6-((1-(2-fluoro-5-methylphenyl)ethyl)amino)-3-(tetrahydro-2H-pyran-4-yl)-1,3,5-triazine-2,4(1H,3H)-dione prepared by the aforementioned methods, with a pharmaceutically acceptable excipient.

The present disclosure also provides use of the crystal forms of (S)-6-((1-(2-fluoro-5-methylphenyl)ethyl)amino)-3-(tetrahydro-2H-pyran-4-yl)-1,3,5-triazine-2,4(1H,3H)-dione or a mixture thereof, or crystal forms prepared by the aforementioned methods or a mixture thereof, or the aforementioned composition, or a composition prepared by the aforementioned method in the preparation of a medicament for treating a disease or disorder associated with myosin regulation.

The present disclosure also provides use of the crystal forms of (S)-6-((1-(2-fluoro-5-methylphenyl)ethyl)amino)-3-(tetrahydro-2H-pyran-4-yl)-1,3,5-triazine-2,4(1H,3H)-dione or a mixture thereof, or crystal forms prepared by the aforementioned methods or a mixture thereof, or the aforementioned composition, or a composition prepared by the aforementioned method in the preparation of a medicament for treating a disease or disorder, the disease or disorder being selected from the group consisting of diastolic heart failure with preserved ejection fraction, ischemic heart disease, angina pectoris, restrictive cardiomyopathy, diastolic dysfunction, hypertrophic cardiomyopathy (HCM), non-obstructive hypertrophic cardiomyopathy (nHCM), obstructive hypertrophic cardiomyopathy (oHCM), valvular diseases, heart failure with preserved ejection fraction (HFpEF), heart failure with mid-range ejection fraction (HFmREF), aortic stenosis, inflammatory cardiomyopathy, Löeffler endocarditis, endomyocardial fibrosis, invasive cardiomyopathy, hemochromatosis, Fabry disease, glycogen storage disease, congenital heart defect, tetralogy of Fallot, left ventricular hypertrophy, refractory angina, and Chagas disease, optionally from the group consisting of ischemic heart disease, restrictive cardiomyopathy, hypertrophic cardiomyopathy (HCM), non-obstructive hypertrophic cardiomyopathy (nHCM), obstructive hypertrophic cardiomyopathy (oHCM), inflammatory cardiomyopathy, invasive cardiomyopathy, congenital heart defect, and left ventricular hypertrophy; optionally, the disease or disorder is hypertrophic cardiomyopathy (HCM).

As used herein, "2θ or 2θ angle" refers to a diffraction angle; θ refers to the Bragg angle in ° or degrees; the 2θ of each characteristic peak has a margin of error of ±0.20 (including the case that a number having more than 1 decimal place is rounded), specifically -0.20, -0.19, -0.18, -0.17, -0.16, - 0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

As used herein, "crystallization" or "crystallizing" includes, but is not limited to, stirring crystallization, triturating crystallization, cooling crystallization, and volatilizing crystallization.

As used herein, "differential scanning calorimetry" or "DSC" refers to the measurement of the temperature difference and heat flow difference between a sample and a reference substance during a ramping or thermostatic process to characterize all the physical changes and chemical changes related to the thermal effect, and to obtain the phase change information about the sample.

As used herein, "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 is an XRD pattern of amorphous (S)-6-((1-(2-fluoro-5-methylphenyl)ethyl)amino)-3-(tetrahydro-2H-pyran-4-yl)-1,3,5-triazine-2,4(1H,3H)-dione.
FIG. 2 is an XRD pattern of a crystal form A of (S)-6-((1-(2-fluoro-5-methylphenyl)ethyl)amino)-3-(tetrahydro-2H-pyran-4-yl)-1,3,5-triazine-2,4(1H,3H)-dione.
FIG. 3 is an XRD pattern of a crystal form B of (S)-6-((1-(2-fluoro-5-methylphenyl)ethyl)amino)-3-(tetrahydro-2H-pyran-4-yl)-1,3,5-triazine-2,4(1H,3H)-dione.
FIG. 4 is an XRD pattern of a crystal form C of (S)-6-((1-(2-fluoro-5-methylphenyl)ethyl)amino)-3-(tetrahydro-2H-pyran-4-yl)-1,3,5-triazine-2,4(1H,3H)-dione.
FIG. 5 is an XRD pattern of a crystal form D of (S)-6-((1-(2-fluoro-5-methylphenyl)ethyl)amino)-3-(tetrahydro-2H-pyran-4-yl)-1,3,5-triazine-2,4(1H,3H)-dione.
FIG. 6 is an XRD pattern of a crystal form E of (S)-6-((1-(2-fluoro-5-methylphenyl)ethyl)amino)-3-(tetrahydro-2H-pyran-4-yl)-1,3,5-triazine-2,4(1H,3H)-dione.

### DETAILED DESCRIPTION

The present disclosure is further illustrated in detail by the following examples and experimental examples. These examples and experimental examples are for illustrative purposes only and are not intended to limit the scope of the present disclosure.

Test conditions for the instruments used in the experiment:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-d6), chloroform-D (CDCl3), and methanol-D4 (CD₃OD) as solvents, and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) and THERMO Ultimate 3000-Q Exactive (manufacturer: THERMO, MS model: THERMO Q Exactive).

The high performance liquid chromatography (HPLC) analyses were performed using Agilent HPLC 1200DAD, Agilent HPLC 1200VWD, and Waters HPLC e2695-2489 high-pressure liquid chromatographs.

The chiral HPLC analyses were performed using an Agilent 1260 DAD high performance liquid chromatograph.

The preparative high performance liquid chromatography was performed using Waters 2767, Waters 2767-SQ Detecor2, Shimadzu LC-20AP, and Gilson-281 preparative chromatographs.

The preparative chiral chromatography was performed using a Shimadzu LC-20AP preparative chromatograph.

The CombiFlash preparative flash chromatograph used was Combiflash Rf200 (TELEDYNE ISCO). The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

In the silica gel column chromatography, a 200-300 mesh silica gel (Huanghai, Yantai) was generally used as the carrier.

The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed in an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 rounds of vacuumization and hydrogen filling. The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The reaction progress monitoring in the examples was performed using thin-layer chromatography (TLC). The developing solvents used in the reactions, the eluent systems used in the column chromatography purification of the compounds, and the developing solvent systems used in the thin-layer chromatography include: A: a n-hexane/ethyl acetate system, and B: a dichloromethane/methanol system. The volume ratio of the solvents was adjusted depending on the polarity of the compounds, or by adding a small amount of basic or acidic reagents such as triethylamine and acetic acid.

When the compounds of the examples contained two or more chiral centers, the relative stereochemistry of these compounds was identified by NMR studies and/or X-ray diffraction. In these cases, the compounds were identified using the prefix "rel" followed by the R/S nomenclature, where R/S provides only relative stereochemical information (for example, trans or cis) and does not indicate absolute stereochemistry.

XRPD refers to X-ray powder diffraction: The measurement was performed using a BRUKER D8 X-ray diffractometer, and the specific acquisition information was: a Cu anode (40 kV, 40 mA), radiation: monochromatic Cu-Kα radiation (λ = 1.5418 Å). The scan mode was: θ/2θ, and the scan range (2θ range) was: 5-50° (or 3-48°).

DSC refers to differential scanning calorimetry: The measurement was performed using a METTLER TOLEDO DSC 3+ differential scanning calorimeter, with a temperature ramp rate of 10 °C/min, specific temperature ranges shown in corresponding patterns (mostly 25-300 °C), and a nitrogen purge speed of 50 mL/min.

TGA refers to thermogravimetric analysis: The measurement was performed using a METTLER TOLEDO TGA 2 thermogravimetric analyzer, with a temperature ramp rate of 10 °C/min, specific temperature ranges shown in corresponding patterns (mostly 25-300 °C), and a nitrogen purge speed of 50 mL/min.

DVS refers to dynamic vapor sorption: The measurement was performed using SMS DVS Advantage; at 25 °C, the humidity was changed as follows: 50%-95%-0%-95%-50%, in steps of 10% (5% for the last step) (specific humidity ranges are shown in corresponding patterns, and those listed here were used in most cases); the criterion was dm/dt being not greater than 0.002%.

### Example 1.

### Preparation of (S)-6-((1-(2-fluoro-5-methylphenyl)ethyl)amino)-3-(tetrahydro-2H-pyran-4-yl)-1,3,5-triazine-2,4(1H,3H)-dione (see the preparation method of Example 16 in Application No. WO2022105852)

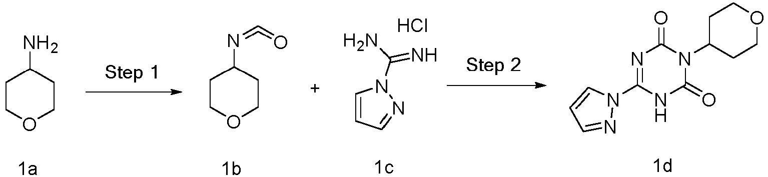

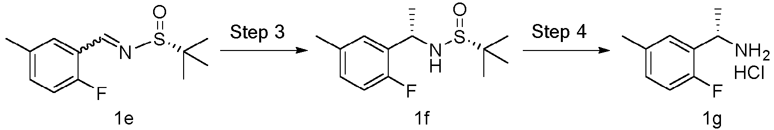

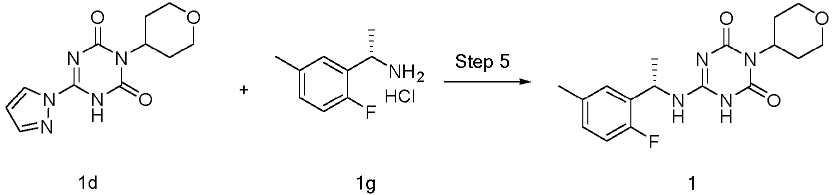

### Step 1

### 4-Isocyanatotetrahydro-2H-pyran 1b

To bis(trichloromethyl)carbonate (11.9 g, 40.0 mmol, Shanghai Titan Scientific Co., Ltd.) in anhydrous dichloromethane (30 mL) at 15 °C was slowly added dropwise a solution of tetrahydro-2*H*-pyran-4-amine **1a** (10.0 g, 100.0 mmol, Shanghai Accela ChemBio Co., Ltd.) and *N,N-*diisopropylethylamine (28.4 g, 220.0 mmol, Shanghai Accela ChemBio Co., Ltd.) in anhydrous dichloromethane (120 mL). The mixture was left to react at room temperature for 2 h. The reaction mixture was concentrated under reduced pressure to give a crude product of the title product **1b.** The crude product was directly used in the next step without purification.

### Step 2

### 6-(1H-Pyrazol-1-yl)-3-(tetrahydro-2H-pyran-4-yl)-1,3,5-triazine-2,4(1H,3H)-dione 1d

To a solution of compound **1c** (10.5 g, 95.2 mmol) and the crude product of compound **1b** in anhydrous *N,N*-dimethylacetamide (120 mL) at -10 °C was slowly added dropwise 1,8-diazabicyclo[5.4.0]undec-7-ene (15.2 g, 100.0 mmol, Shanghai Accela ChemBio Co., Ltd.). The reaction was stirred at 0 °C for 1 h. Subsequently, carbonyldiimidazole (23.2 g, 142.8 mmol, Bide Pharmatech Ltd.) and 1,8-diazabicyclo[5.4.0]undec-7-ene (21.7 g, 142.8 mmol, Shanghai Accela ChemBio Co., Ltd.) were added to the reaction at 0 °C. The reaction was stirred at 60 °C for 16 h. The reaction mixture was concentrated under reduced pressure, and dichloromethane was added to the resulting residue. The mixture was stirred and filtered, and the filter cake was collected and dried under reduced pressure to give the title product **1d** (16.6 g, two-step yield: 63.4%). MS m/z (ESI): 264.1 [M+1].

### Step 3

### (R)-N-((S)-1-(2-Fluoro-5-methylphenyl)ethyl)-2-methylpropane-2-sulfinamide 1f

(*R*)-*N*-(2-Fluoro-5-methylbenzylidene)-2-methylpropane-2-sulfinamide **1e** (6.90 g, 28.59 mmol, prepared by "the method for intermediate 3B disclosed on page 56 of the specification in the patent application WO2020092208A1") was dissolved in dichloromethane (100 mL), and the system was purged with nitrogen three times. The reaction was cooled to -60 °C, and a 3 M solution of methylmagnesium bromide in 2-methyltetrahydrofuran (19.1 mL, 57.18 mmol) was added dropwise. The reaction was stirred at room temperature for 2 h in a nitrogen atmosphere. A saturated ammonium chloride solution (100 mL) was added, and the organic phase was separated. The aqueous phase was extracted with ethyl acetate (80 mL × 2). The organic phases were combined, washed with a saturated sodium chloride solution (100 mL), dried over anhydrous sodium sulfate, filtered, concentrated under reduced pressure, and purified by high performance liquid chromatography (Boston Phlex C18 150 × 30 mm, 5 µm; elution system: water (10 mmol of ammonium bicarbonate), acetonitrile, 20-95% acetonitrile; 20 min of gradient elution; flow rate: 30 mL/min) to give the title product **1f** (5.60 g, yield: 76.1%). MS m/z (ESI): 258.0 [M+1].

### Step 4

### (S)-1-(2-Fluoro-5-methylphenyl)ethylamine hydrochloride 1g

Compound **1f** (670 mg, 2.60 mmol) was dissolved in ethanol (10 mL). The solution was cooled to 0 °C, and thionyl chloride (620 mg, 5.21 mmol) was added dropwise. The reaction was stirred at room temperature for 1 h. The reaction mixture was concentrated under reduced pressure to give a crude product of the title product **1g** (493 mg, yield: 99.9%). The crude product was directly used in the next step without purification. MS m/z (ESI): 153.9 [M+1].

### Step 5

### (S)-6-((1-(2-Fluoro-5-methylphenyl)ethyl)amino)-3-(tetrahydro-2H-pyran-4-yl)-1,3,5-triazine-2,4(1H,3H)-dione 1

Compound **1d** (425 mg, 1.61 mmol) and compound **1g** (297 mg, 1.94 mmol) were dissolved in 1,4-dioxane (10 mL), and the reaction was stirred at 120 °C for 16 h. The reaction mixture was concentrated under reduced pressure and purified by high performance liquid chromatography (Boston Phlex C18 150 × 30 mm, 5 µm; elution system: water (10 mmol of ammonium bicarbonate), acetonitrile, 20-95% acetonitrile; 20 min of gradient elution; flow rate: 30 mL/min) to give the product 1 (310 mg, yield: 55.1%). MS m/z (ESI): 349.0 [M+1].

¹H NMR (500 MHz, DMSO-*d*₆) δ 10.50 (brs, 1H), 7.28-7.05 (m, 4H), 5.22 (m, 1H), 4.64 (m, 1H), 3.90-3.86 (m, 2H), 3.33-3.27 (m, 2H), 2.54-2.42 (m, 2H), 2.28 (s, 3H), 1.45-1.39 (m, 5H).

### Example 2. Preparation of Amorphous (S)-6-((1-(2-Fluoro-5-methylphenyl)ethyl) amino)-3-(tetrahydro-2H-pyran-4-yl)-1,3,5-triazine-2,4(1H,3H)-dione

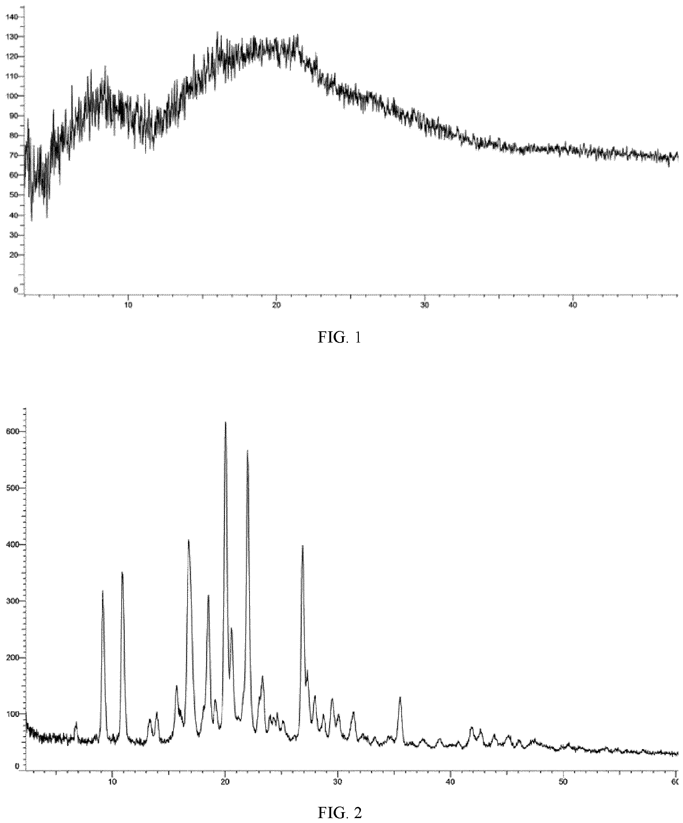

The substance obtained from the high performance liquid chromatography purification in Example 1 was lyophilized to give a product. The product was identified by X-ray powder diffraction as amorphous (S)-6-((1-(2-fluoro-5-methylphenyl)ethyl)amino)-3-(tetrahydro-2H-pyran-4-yl)-1,3,5-triazine-2,4(1H,3H)-dione. An X-ray powder diffraction pattern represented by 2θ angles, which are diffraction angles, has no characteristic peak in the range of 3-48°. The XRD pattern is shown in FIG. 1.

### Example 3. Inhibitory Effect of (S)-6-((1-(2-Fluoro-5-methylphenyl)ethyl)amino)-3-(tetrahydro-2H-pyran-4-yl)-1,3,5-triazine-2,4(1H,3H)-dione on Activity of Myosin ATPase

The method below was used to measure the inhibitory effect of the compound on the activity of myosin ATPase. The experimental method is briefly described below:
1. Experimental materials and instruments
   a. Myocardial actin (Cytoskeleton, AD99)
   b. Myosin motor protein S1 fragment (Cytoskeleton, CS-MYS03)
   c. ATP (Sigma, A7699-1G)
   d. UltraPureTM 1 M Tris-HCl buffer, pH 7.5 (Thermo, 15567027)
   e. CytoPhosTM phosphate assay biological kit (Cytoskeleton, BK054)
   f. Magnesium chloride solution (Sigma, 68475-100ML-F)
   g. Potassium chloride solution (Sigma, 60142-100ML-F)
   h. EGTA (Sigma, E3889-100G)
   i. 96-well plates (Corning 3697)
   j. U-shaped bottom 96-well plates (Corning, 3795)
   k. Microplate reader (BMG, PHERAstar)
   l. Thermostatic incubator (BOXUN, SPX-100B-Z)
2. Procedures

1.61 µM myocardial actin and 0.07 µM myosin motor protein S1 fragment were mixed with different concentrations of the small-molecule compound (initial concentration of 100 µM, serially diluted 3-fold to obtain 9 concentrations), and the plate was incubated at 37 °C for 1 h. Then 120 µM ATP was added, and the plate was incubated at 37 °C for 2 h. Finally, the assay solution in the CytoPhosTM phosphate assay biological kit was added to each well (70 µL/well), and the plate was incubated at room temperature for 10 min. The OD values at the wavelength of 650 nM were measured using the microplate reader. The Pi amount was calculated according to the standard curve. The data were processed using GraphPad software. An inhibition curve was plotted according to the compound concentrations and the corresponding inhibition rates, and the compound concentration at which the inhibition rate was 50%, i.e., the IC₅₀ value, was calculated.

Conclusion: The IC₅₀ for the inhibitory activity of (S)-6-((1-(2-fluoro-5-methylphenyl)ethyl)amino)-3-(tetrahydro-2H-pyran-4-yl)-1,3,5-triazine-2,4(1H,3H)-dione against myosin ATPase is 1.06 µM, indicating that the compound has a very good inhibitory effect on myosin ATPase.

### Example 4. Toxicokinetic Evaluation of 14 Days of Repeated Intragastric Administration of (S)-6-((1-(2-Fluoro-5-methylphenyl)ethyl)amino)-3-(tetrahydro-2H-pyran-4-yl)-1,3,5-triazine-2,4(1H,3H)-dione to SD Rats

### 1. Abstract

SD rats were used as the test animals. After intragastric administration of the test compounds to the SD rats, the concentrations of the original forms of the compounds in the plasma and the administration solutions were measured by the LC/MS/MS method at different time points. The toxicokinetic behavior of (S)-6-((1-(2-fluoro-5-methylphenyl)ethyl)amino)-3-(tetrahydro-2H-pyran-4-yl)-1,3,5-triazine-2,4(1H,3H)-dione in the SD rats was studied, and its toxicokinetic characteristics were evaluated.

### 2. Experimental protocol

### 2.1. Test compounds

### (S)-6-((1-(2-Fluoro-5-methylphenyl)ethyl)amino)-3-(tetrahydro-2H-pyran-4-yl)-1,3,S-triazine-

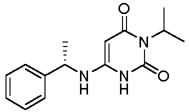

### 2,4(1H,3H)-dione and compound MYK-461 (MYK-461, Example 1 of WO2014205223A1).

### 2.2. Experimental animals

Twenty-four SD rats, with an equal number of males and females, were evenly divided into 6 groups, each consisting of 4 rats with an equal number of males and females. The rats were provided by Vital River Laboratory Animal Technology Co., Ltd.

### 2.3. Compound solution preparation

A certain amount of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 15% PEG400 and 85% (10% TPGS + 1% HPMC K100LV) was added to prepare a pale yellow homogeneous suspension.

A certain amount of compound MYK-461 was weighed out, and 0.5% MC was added to prepare a colorless clear solution.

### 2.4. Administration

(S)-6-((1-(2-Fluoro-5-methylphenyl)ethyl)amino)-3-(tetrahydro-2H-pyran-4-yl)-1,3,5-triazine-2,4(1H,3H)-dione was intragastrically administered at doses of 5 mg/kg, 15 mg/kg, and 30 mg/kg, each with a volume of 10 mL/kg. Compound MYK-461 was administered at doses of 0.5 mg/kg, 1.5 mg/kg, and 3 mg/kg, each with a volume of 10 mL/kg.

### 3. Procedures

0.2-mL blood samples were collected from the orbit at 0.5 h, 1.0 h, 2.0 h, 4.0 h, 8.0 h, and 24.0 h after administration on day 1, and 0.2-mL blood samples were collected from the orbit before administration and at 0.5 h, 1.0 h, 2.0 h, 4.0 h, 8.0 h, and 24.0 h after administration on day 7 and day 14. The blood samples were placed into EDTA-K2 anticoagulation tubes and centrifuged at 10,000 rpm for 1 min (4 °C), and the plasma was isolated within 1 h and stored at -20 °C before analysis. The procedure starting from the blood collection to the centrifugation was performed under ice-bath conditions. Two hours after administration, food was provided.

Determination of the plasma concentrations of the test compounds in SD rats after intragastric administration of different concentrations of the compounds: 20 µL of the SD rat plasma at each time point after administration was taken, and 50 µL of an internal standard solution (internal standard for (S)-6-((1-(2-fluoro-5-methylphenyl)ethyl)

amino)-3-(tetrahydro-2H-pyran-4-yl)-1,3,5-triazine-2,4(1H,3H)-dione: 100 ng/mL verapamil; internal standard for compound MYK-461: 100 ng/mL camptothecin) and 200 µL of acetonitrile were added; the mixture was vortexed for 5 min and centrifuged at 3700 rpm for 10 min, and 1 µL of the supernatant was taken for LC/MS/MS analysis.

### 4. Toxicokinetic parameter results

**Table 1. The pharmacokinetic parameters of the compounds in SD rats**

| Compound | Dose (mg/kg) | Date | Plasma concentration | Area under curve | Half-life | Residence time | Clearance rate | Apparent volume of distribution |
|---|---|---|---|---|---|---|---|---|
| | | | Cmax (ng/mL) | AUC (ng/mL*h) | T_{1/2} (h) | MRT (h) | CL/F (mL/min/kg) | Vz/F (mL/kg) |
| (S)-6-((1-(2-Fluoro-5-methylphenyl) ethyl)amino)-3-(tetrahydro-2H-pyran-4-yl)-1,3,5-triazine-2,4(1H,3H)-dione | *5* | Day 1 | 5735 | 30026 | 4.8 | *5.0* | 3.06 | 1265 |
| | | Day 7 | 4473 | 28136 | 4.9 | 6.0 | 2.99 | 1279 |
| | | Day 14 | 4858 | 29780 | 5.1 | 6.2 | 2.80 | 1239 |
| | 15 | Day 1 | 16125 | 80443 | 5.1 | 5.4 | 3.08 | 1369 |
| | | Day 7 | 16800 | 70916 | 6.5 | 6.7 | 3.58 | 2129 |
| | | Day 14 | 14125 | 82906 | 5.6 | 6.4 | 2.98 | 1445 |
| | 30 | Day 1 | 18725 | 104640 | 5.6 | 6.4 | 4.72 | 2257 |
| | | Day 7 | 21550 | 120552 | 5.9 | 6.7 | 4.14 | 2146 |
| | | Day 14 | 20000 | 139432 | 5.6 | 6.9 | 3.52 | 1689 |
| MYK -461 | *0.5* | Day 1 | 40.2 | 491 | 13.3 | 19.0 | 12.9 | 13778 |
| | | Day 7 | 51.7 | 888 | 14.7 | 21.3 | 7.46 | 8412 |
| | | Day 14 | 89.3 | 1558 | 32.9 | 47.3 | 3.06 | 5861 |
| | 1.5 | Day 1 | 140 | 2577 | 19.1 | 28.2 | 6.39 | 9561 |
| | | Day 7 | 269 | 5181 | 20.5 | 30.2 | 3.58 | 4802 |
| | | Day 14 | 333 | 6320 | 26.2 | 38.5 | 2.57 | 5027 |
| | 3.0 | Day 1 | 297 | 5213 | 28.5 | 43.1 | 4.91 | 8931 |
| | | Day 7 | 628 | 12549 | 190 | 275 | 1.97 | 4355 |
| | | Day 14 | 767 | 15985 | 46.5 | 68.1 | 1.3 | 3802 |

Conclusion: The 14 days of repeated intragastric administration of (S)-6-((1-(2-fluoro-5-methylphenyl)ethyl)amino)-3-(tetrahydro-2H-pyran-4-yl)-1,3,5-triazine-2,4(1H,3H)-dione to SD rats did not cause significant accumulation in the SD rats, whereas the administration of compound MYK-461 caused significant accumulation in the SD rats, increasing the medication risk. Apparently, (S)-6-((1-(2-fluoro-5-methylphenyl)ethyl)amino)-3-(tetrahydro-2H-pyran-4-yl)-1,3,5-triazine-2,4(1H,3H)-dione has a significant toxicokinetic advantage over compound MYK-461.

### Example 5. Preparation of Crystal Form A of Compound

300 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 6 mL of 7% water/ethanol was added. The compound was completely dissolved by heating at 80 °C, and the solution was cooled to room temperature and then stirred overnight. After filtration, the solid was dried *in vacuo* to give a product. The product was identified by X-ray powder diffraction as the crystal form A. The X-ray powder diffraction data are shown in Table 2, and an X-ray powder diffraction pattern is shown in FIG. 2.

A DSC profile shows that: the peak value of the endothermic peak is 256.71 °C. A TGA profile shows a weight loss of 0.59% from 30 °C to 100 °C.

DVS testing shows that the sample had a hygroscopic weight gain of about 0.12% under normal storage conditions (i.e., room temperature and 60% RH), a hygroscopic weight gain of about 0.15% under accelerated experimental conditions (i.e., 70% RH), and a hygroscopic weight gain of about 0.24% under extreme conditions (i.e., 90% RH). Moreover, according to another measurement, the crystal form did not change after DVS testing.

**Table 2. The peak positions of the crystal form A of the compound**

| Peak No. | 2θ [° or degrees] | d [Å] | Relative intensity (%) |
|---|---|---|---|
| 1 | 6.792 | 13.00289 | 5.5 |
| 2 | 9.167 | 9.63949 | 48.8 |
| 3 | 10.928 | 8.08983 | 54.5 |
| 4 | 13.325 | 6.63932 | 6.9 |
| 5 | 13.947 | 6.34463 | 8.6 |
| 6 | 15.714 | 5.63498 | 15.9 |
| 7 | 16.084 | 5.50611 | 7.8 |
| 8 | 16.803 | 5.27216 | 62.9 |
| 9 | 18.524 | 4.78597 | 44.1 |
| 10 | 19.144 | 4.63244 | 8.1 |
| 11 | 20.056 | 4.42380 | 100.0 |
| 12 | 20.585 | 4.31129 | 31.4 |
| 13 | 22.011 | 4.03511 | 91.5 |
| 14 | 23.318 | 3.81170 | 17.4 |
| 15 | 23.996 | 3.70548 | 4.3 |
| 16 | 24.320 | 3.65695 | 4.0 |
| 17 | 24.647 | 3.60916 | 5.7 |
| 18 | 25.176 | 3.53441 | 3.5 |
| 19 | 26.880 | 3.31418 | 62.9 |
| 20 | 27.317 | 3.26209 | 20.9 |
| 21 | 27.945 | 3.19025 | 11.8 |
| 22 | 28.718 | 3.10611 | 5.9 |
| 23 | 29.501 | 3.02545 | 11.5 |
| 24 | 30.062 | 2.97022 | 6.1 |
| 25 | 31.399 | 2.84670 | 8.3 |
| 26 | 32.248 | 2.77368 | 2.1 |
| 27 | 33.300 | 2.68845 | 1.5 |
| 28 | 34.575 | 2.59214 | 0.6 |
| 29 | 35.518 | 2.52542 | 14.3 |
| 30 | 37.552 | 2.39323 | 1.6 |
| 31 | 39.031 | 2.30587 | 1.6 |
| 32 | 40.744 | 2.21275 | 0.7 |
| 33 | 41.874 | 2.15565 | 5.7 |
| 34 | 42.664 | 2.11753 | 4.7 |
| 35 | 43.847 | 2.06310 | 2.5 |
| 36 | 45.128 | 2.00748 | 1.8 |
| 37 | 46.066 | 1.96876 | 1.2 |
| 38 | 47.297 | 1.92034 | 1.0 |

### Example 6. Preparation of Crystal Form A of Compound

(S)-6-((1-(2-Fluoro-5-methylphenyl)ethyl)amino)-3-(tetrahydro-2H-pyran-4-yl)-1,3,5-triazine-2,4(1H,3H)-dione was added to a solvent and triturated. After centrifugation, the solid was dried *in vacuo* to give a product. The crystal form was determined by X-ray powder diffraction, as shown in Table 3.

**Table 3. Preparation of crystal form A by triturating crystallization**

| No. | Feed amount and solvent | Crystal form |
|---|---|---|
| 1 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of water, and triturating at room temperature for 4 days | A |
| 2 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of methanol, and triturating at room temperature for 4 days | A |
| 3 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of ethanol, and triturating at room temperature for 4 days | A |
| 4 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of isopropanol, and triturating at room temperature for 4 days | A |
| 5 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of acetone, and triturating at room temperature for 4 days | A |
| 6 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of ethyl acetate, and triturating at room temperature for 4 days | A |
| 7 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of acetonitrile, and triturating at room temperature for 4 days | A |
| 8 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of isopropyl acetate, and triturating at room temperature for 4 days | A |
| 9 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of methyl tert-butyl ether, and triturating at room temperature for 4 days | A |
| 10 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of methyl isobutyl ketone, and triturating at room temperature for 4 days | A |
| 11 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of n-heptane, and triturating at room temperature for 4 days | A |
| 12 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of cyclohexane, and triturating at room temperature for 4 days | A |
| 13 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of 1,4-dioxane, and triturating at room temperature for 4 days | A |
| 14 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of 10% water/methanol, and triturating at room temperature for 4 days | A |
| 15 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of 7% water/ethanol, and triturating at room temperature for 4 days | A |

### Example 7. Preparation of Crystal Form A of Compound

(S)-6-((1-(2-Fluoro-5-methylphenyl)ethyl)amino)-3-(tetrahydro-2H-pyran-4-yl)-1,3,5-triazine-2,4(1H,3H)-dione was added to a solvent and triturated. After centrifugation, the solid was dried *in vacuo* to give a product. The crystal form was determined by X-ray powder diffraction, as shown in Table 4.

**Table 4. Preparation of crystal form A by triturating crystallization**

| No. | Feed amount and solvent | Crystal form |
|---|---|---|
| 1 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of water, and triturating at 50 °C for 1 day | A |
| 2 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of isopropanol, and triturating at 50 °C for 1 day | A |
| 3 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of acetone, and triturating at 50 °C for 1 day | A |
| 4 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of ethyl acetate, and triturating at 50 °C for 1 day | A |
| 5 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of acetonitrile, and triturating at 50 °C for 1 day | A |
| 6 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of isopropyl acetate, and triturating at 50 °C for 1 day | A |
| 7 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of methyl tert-butyl ether, and triturating at 50 °C for 1 day | A |
| 8 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of methyl isobutyl ketone, and triturating at 50 °C for 1 day | A |
| 9 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of n-heptane, and triturating at 50 °C for 1 day | A |
| 10 | Weighing out 10 mg of (S)-6-((1-(2-fluoro-5-methylphenyl)ethyl)amino)-3-(tetrahydro-2H-pyran-4-yl)-1,3,5-triazine-2,4(1H,3H)-dione, adding 1 mL of cyclohexane, and triturating at 50 °C for 1 day | A |

### Example 8. Preparation of Crystal Form A of Compound

10 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.1 mL of tetrahydrofuran was added. The compound was completely dissolved by stirring, and volatilizing crystallization was performed to give a product. The product was identified by X-ray powder diffraction as the crystal form A.

### Example 9. Preparation of Crystal Form A of Compound

10 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 1 mL of 10% water/isopropanol was added. The compound was completely dissolved by stirring, and volatilizing crystallization was performed to give a product. The product was identified by X-ray powder diffraction as the crystal form A.

### Example 10. Preparation of Crystal Form A of Compound

10 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 1 mL of 10% water/acetone was added. The compound was completely dissolved by stirring, and volatilizing crystallization was performed to give a product. The product was identified by X-ray powder diffraction as the crystal form A.

### Example 11. Preparation of Crystal Form A of Compound

10 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 1 mL of methanol was added. The compound was completely dissolved by stirring at 50 °C, and cooling crystallization was performed to give a product. The product was identified by X-ray powder diffraction as the crystal form A.

### Example 12. Preparation of Crystal Form A of Compound

10 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 1 mL of ethanol was added. The compound was completely dissolved by stirring at 50 °C, and cooling crystallization was performed to give a product. The product was identified by X-ray powder diffraction as the crystal form A.

### Example 13. Preparation of Crystal Form A of Compound

10 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 1 mL of 10% water/methanol was added. The compound was completely dissolved by stirring at 50 °C, and cooling crystallization was performed to give a product. The product was identified by X-ray powder diffraction as the crystal form A.

### Example 14. Preparation of Crystal Form A of Compound

10 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 1 mL of 7% water/ethanol was added. The compound was completely dissolved by stirring at 50 °C, and cooling crystallization was performed to give a product. The product was identified by X-ray powder diffraction as the crystal form A.

### Example 15. Preparation of Crystal Form A of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of methanol was added. The mixture was stirred, and trituration was performed at room temperature for 3 days. After centrifugation, the solid was dried *in vacuo* to give a product. The product was identified by X-ray powder diffraction as the crystal form A.

### Example 16. Preparation of Crystal Form A of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of ethanol was added. The mixture was stirred, and trituration was performed at room temperature for 3 days. After centrifugation, the solid was dried *in vacuo* to give a product. The product was identified by X-ray powder diffraction as the crystal form A.

### Example 17. Preparation of Crystal Form A of Compound

1 g of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 30 mL of 10% water/acetone was added. The compound was completely dissolved by stirring at 50 °C, and 30 mL of water was added. The mixture was stirred at 50 °C for 5 h, cooled to 25 °C, and stirred overnight. After filtration, the solid was dried *in vacuo* to give a product. The product was identified by X-ray powder diffraction as the crystal form A.

### Example 18. Preparation of Crystal Form B of Compound

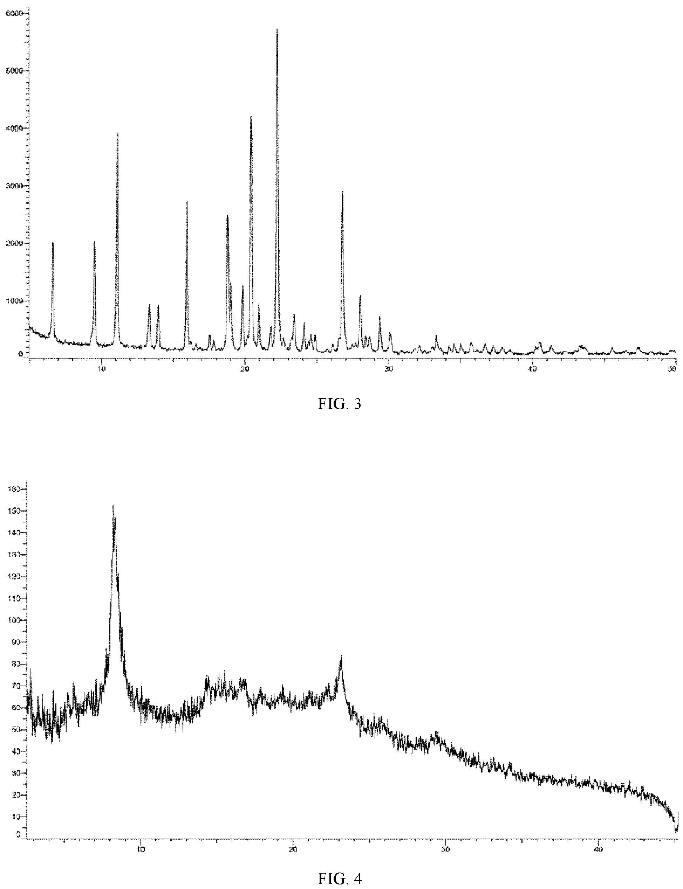

5 g of (S)-6-((1-(2-fluoro-5-methylphenyl)ethyl)amino)-3-(tetrahydro-2H-pyran-4-yl)-1,3,5-triazine-2,4(1H,3H)-dione was dissolved in 250 mL of absolute ethanol. The mixture was heated at reflux, and the solid was completely dissolved. The heating was stopped, and the mixture was left to stand and filtered. The filter cake was collected and dried to give a product. The product was identified by X-ray powder diffraction as the crystal form B. The X-ray powder diffraction data are shown in Table 5, and an X-ray powder diffraction pattern is shown in FIG. 3.

A DSC profile shows that: the peak value of the endothermic peak is 256.45 °C. A TGA profile shows that: the compound had a weight loss of 1.32% from 40 °C to 190 °C. DVS experimental data show that the sample had a hygroscopic weight gain of about 0.242% under normal storage conditions (i.e., 25 °C and 60% RH), a hygroscopic weight gain of about 0.298% under accelerated test conditions (i.e., 70% RH), and a hygroscopic weight gain of about 0.418% under extreme conditions (90% RH). The XRD pattern shows that the crystal form did not change before and after DVS testing.

**Table 5. The peak positions of the crystal form B of the compound**

| Peak No. | 2θ value [° or degrees] | D[Å] | Relative intensity (%) |
|---|---|---|---|
| 1 | 6.638 | 13.30516 | 30.3 |
| 2 | 9.522 | 9.28101 | 32.7 |
| 3 | 11.108 | 7.9588 | 66.5 |
| 4 | 13.342 | 6.63115 | 13.4 |
| 5 | 13.967 | 6.33549 | 13.3 |
| 6 | 15.949 | 5.55235 | 45.8 |
| 7 | 17.535 | 5.05373 | 4.7 |
| 8 | 17.827 | 4.97144 | 3.2 |
| 9 | 18.788 | 4.71925 | 41.8 |
| 10 | 19.026 | 4.66084 | 21.1 |
| 11 | 19.847 | 4.46990 | 19.8 |
| 12 | 20.429 | 4.34376 | 72.3 |
| 13 | 20.968 | 4.23329 | 14.2 |
| 14 | 21.783 | 4.07672 | 7.0 |
| 15 | 22.24 | 3.99394 | 100.0 |
| 16 | 22.692 | 3.91537 | 3.5 |
| 17 | 23.415 | 3.79623 | 10.7 |
| 18 | 24.096 | 3.69041 | 8.8 |
| 19 | 24.579 | 3.61895 | 5.4 |
| 20 | 24.87 | 3.57729 | 5.2 |
| 21 | 26.78 | 3.32635 | 50.0 |
| 22 | 28.023 | 3.18149 | 17.3 |
| 23 | 28.425 | 3.13742 | 4.5 |
| 24 | 28.679 | 3.11017 | 4.5 |
| 25 | 29.373 | 3.03827 | 11.3 |
| 26 | 30.093 | 2.96723 | 5.5 |
| 27 | 33.317 | 2.68706 | 5.4 |
| 28 | 35.733 | 2.51074 | 3.1 |
| 29 | 40.517 | 2.22467 | 3.4 |

### Example 19. Preparation of Crystal Form B of Compound

30 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.3 mL of dichloromethane was added. The compound was completely dissolved by stirring at room temperature, and 2.1 mL of ethyl acetate was added. The mixture was stirred at room temperature for 2 days. After filtration, the solid was dried *in vacuo* to give a product. The product was identified by X-ray powder diffraction as the crystal form B.

### Example 20. Preparation of Crystal Form B of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of acetone was added. The mixture was stirred, and trituration was performed at room temperature for 3 days. After centrifugation, the solid was dried *in vacuo* to give a product. The product was identified by X-ray powder diffraction as the crystal form B.

### Example 21. Preparation of Crystal Form B of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of ethyl acetate was added. The mixture was stirred, and trituration was performed at room temperature for 3 days. After centrifugation, the solid was dried *in vacuo* to give a product. The product was identified by X-ray powder diffraction as the crystal form B.

### Example 22. Preparation of Crystal Form B of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of acetonitrile was added. The mixture was stirred, and trituration was performed at room temperature for 3 days. After centrifugation, the solid was dried *in vacuo* to give a product. The product was identified by X-ray powder diffraction as the crystal form B.

### Example 23. Preparation of Crystal Form B of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of methyl isobutyl ketone was added. The mixture was stirred, and trituration was performed at room temperature for 3 days. After centrifugation, the solid was dried *in vacuo* to give a product. The product was identified by X-ray powder diffraction as the crystal form B.

### Example 24. Preparation of Crystal Form C of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of isopropyl acetate was added. The mixture was stirred for crystallization, and trituration was performed at room temperature for 3 days. After centrifugation, the solid was dried *in vacuo* to give the title product. The product was identified by X-ray powder diffraction as the crystal form C. An XRD pattern is shown in FIG. 4, and its characteristic peak positions are shown in Table 6. A DSC profile shows that the peak values of the endothermic peaks are 86.45 °C and 255.56 °C, and the peak value of the exothermic peak is 194.44 °C. A TGA profile shows a weight loss of 5.17% from 30 °C to 140 °C.

**Table 6. The peak positions of the crystal form C of the compound**

| Peak No. | 2θ [° or degrees] | d [Å] | Relative intensity (%) |
|---|---|---|---|
| 1 | 8.336 | 10.59832 | 100.0 |
| 2 | 14.395 | 6.14797 | 6.4 |
| 3 | 16.750 | 5.28863 | 9.1 |
| 4 | 17.834 | 4.96943 | 4.2 |
| 5 | 19.291 | 4.59747 | 3.6 |
| 6 | 22.327 | 3.97863 | 5.4 |
| 7 | 23.070 | 3.85219 | 23.6 |

### Example 25. Preparation of Crystal Form D of Compound

5 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.05 mL of dichloromethane was added. The compound was completely dissolved by stirring at room temperature, and 0.35 mL of methyl tert-butyl ether was added. The mixture was stirred for crystallization, and trituration was performed at room temperature for 3 days. After centrifugation, the solid was dried *in vacuo* to give a product. The product was identified by X-ray powder diffraction as the crystal form D. An XRD pattern is shown in FIG. 4, and its characteristic peak positions are shown in Table 7. A DSC profile shows that the peak values of the endothermic peaks are 96.46 °C and 256.21 °C, and the peak value of the exothermic peak is 160.48 °C. A TGA profile shows a weight loss of 9.49% from 30 °C to 140 °C.

**Table 7. The peak positions of the crystal form D of the compound**

| Peak No. | 2θ [° or degrees] | d [Å] | Relative intensity (%) |
|---|---|---|---|
| 1 | 6.969 | 12.67472 | 7.6 |
| 2 | 7.778 | 11.35735 | 70.2 |
| 3 | 8.651 | 10.21361 | 100.0 |
| 4 | 9.662 | 9.14614 | 36.7 |
| 5 | 13.469 | 6.56865 | 19.4 |
| 6 | 13.965 | 6.33638 | 23.9 |
| 7 | 15.004 | 5.89981 | 7.3 |
| 8 | 15.488 | 5.71645 | 14.7 |
| 9 | 16.681 | 5.31042 | 16.3 |
| 10 | 17.484 | 5.06818 | 14.4 |
| 11 | 18.114 | 4.89333 | 2.9 |
| 12 | 19.588 | 4.52839 | 18.9 |
| 13 | 20.173 | 4.39837 | 17.6 |
| 14 | 20.860 | 4.25502 | 5.9 |
| 15 | 21.311 | 4.16599 | 8.6 |
| 16 | 22.938 | 3.87398 | 23.1 |
| 17 | 23.931 | 3.71540 | 13.0 |
| 18 | 25.200 | 3.53117 | 9.9 |
| 19 | 26.241 | 3.39334 | 1.6 |
| 20 | 27.604 | 3.22882 | 5.5 |
| 21 | 29.829 | 2.99291 | 8.9 |
| 22 | 32.620 | 2.74289 | 2.8 |

### Example 26. Preparation of Crystal Form E of Compound

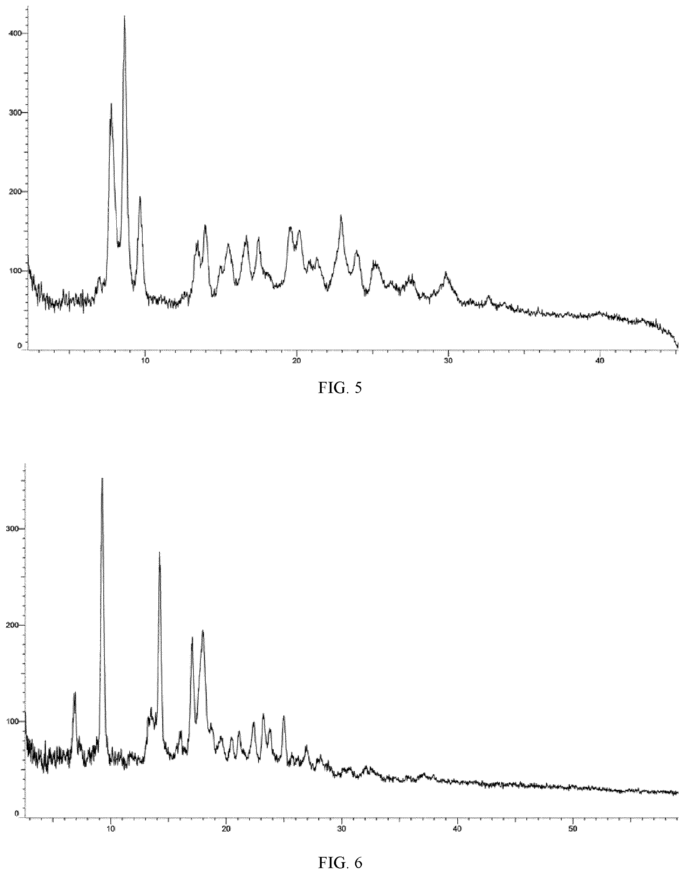

30 mg of (S)-6-((1-(2-fluoro-5-methylphenyl)ethyl)amino)-3-(tetrahydro-2H-pyran-4-yl)-1,3,5-triazine-2,4(1H,3H)-dione was weighed out, and 0.15 mL of dichloromethane was added. The compound was completely dissolved by stirring at room temperature, and 3.15 mL of n-heptane was added. The mixture was stirred for crystallization, and trituration was performed at room temperature for 2 days. After centrifugation, the solid was dried *in vacuo* to give a product. The product was identified by X-ray powder diffraction as the crystal form E. An XRD pattern is shown in FIG. 5, and its characteristic peak positions are shown in Table 8. A DSC profile shows that the peak values of the endothermic peaks are 104.97 °C, 128.14 °C, 205.60 °C, 231.49 °C, and 254.86 °C, and the peak value of the exothermic peak is 184.66 °C. A TGA profile shows a weight loss of 0.86% from 30 °C to 100 °C.

**Table 8. The peak positions of the crystal form E of the compound**

| Peak No. | 2θ [° or degrees] | d [Å] | Relative intensity (%) |
|---|---|---|---|
| 1 | 6.934 | 12.73692 | 24.2 |
| 2 | 9.264 | 9.53856 | 100.0 |
| 3 | 13.241 | 6.68135 | 12.7 |
| 4 | 13.485 | 6.56088 | 13.7 |
| 5 | 14.249 | 6.21093 | 68.7 |
| 6 | 15.990 | 5.53823 | 6.3 |
| 7 | 16.092 | 5.50344 | 6.4 |
| 8 | 17.050 | 5.19617 | 38.2 |
| 9 | 17.993 | 4.92605 | 40.4 |
| 10 | 18.753 | 4.72799 | 6.1 |
| 11 | 19.570 | 4.53254 | 4.4 |
| 12 | 20.449 | 4.33959 | 4.8 |
| 13 | 21.098 | 4.20754 | 9.1 |
| 14 | 22.356 | 3.97354 | 10.8 |
| 15 | 23.209 | 3.82931 | 13.8 |
| 16 | 23.774 | 3.73957 | 8.3 |
| 17 | 24.993 | 3.55997 | 14.9 |
| 18 | 26.939 | 3.30705 | 4.1 |
| 19 | 28.153 | 3.16711 | 3.9 |

### Experimental Example 1. Influencing Factor Stability Study of Crystal Form A of Compound

The crystal form A of (S)-6-((1-(2-fluoro-5-methylphenyl)ethyl)amino)-3-(tetrahydro-2H-pyran-4-yl)-1,3,5-triazine-2,4(1H,3H)-dione was spread out and left to stand in open flasks, and the samples were subjected to 30-day stability tests under high temperature (40 °C and 60 °C), light exposure, and high humidity (RH 75% and RH 92.5%) conditions.

**Table 9. The influencing factor stability of the crystal form A of the compound**

| Conditions | Time (days) | Color and state | Purity% | Crystal form |
|---|---|---|---|---|
| Initial | 0 | Off-white solid | 99.7 | Crystal form A |
| Light exposure (4500 Lux) | 5 | Off-white solid | 99.7 | No change |
| | 10 | Off-white solid | 99.7 | No change |
| | 30 | Off-white solid | 99.7 | No change |
| 40 °C | 5 | Off-white solid | 99.7 | No change |
| | 10 | Off-white solid | 99.7 | No change |
| | 30 | Off-white solid | 99.7 | No change |
| 60 °C | 5 | Off-white solid | 99.7 | No change |
| | 10 | Off-white solid | 99.7 | No change |
| | 30 | Off-white solid | 99.7 | No change |
| 75% RH | 5 | Off-white solid | 99.7 | No change |
| | 10 | Off-white solid | 99.7 | No change |
| | 30 | Off-white solid | 99.7 | No change |
| 92.5% RH | 5 | Off-white solid | 99.7 | No change |
| | 10 | Off-white solid | 99.7 | No change |
| | 30 | Off-white solid | 99.7 | No change |

Conclusion: The influencing factor experiments show that: standing under light exposure, high temperature (40 °C and 60 °C), and high humidity (75% RH and 92.5% RH) conditions for 30 days, the free-state crystal form A exhibited both good physical stability and good chemical stability.

Experimental Example 2. Long-Term/Accelerated Stability Study of Crystal Form A of Compound The crystal form A of (S)-6-((1-(2-fluoro-5-methylphenyl)ethyl)amino)-3-(tetrahydro-2H-pyran-4-yl)-1,3,5-triazine-2,4(1H,3H)-dione was subjected to 6-month long-term accelerated stability tests under long-term (25 °C, 60% RH) and accelerated (40 °C, 75% RH) conditions.

**Table 10. The long-term accelerated stability of the crystal form A of the compound**

| Test conditions | Time (months) | Purity (%) | Crystal form |
|---|---|---|---|
| Initial | | 99.7 | Crystal form A |
| 25 °C/60% RH | 1 | 99.7 | No change |
| | 2 | 99.7 | No change |
| | 3 | 99.7 | No change |
| | 6 | 99.7 | No change |
| 40 °C/75% RH | 1 | 99.7 | No change |
| | 2 | 99.7 | No change |
| | 3 | 99.7 | No change |
| | 6 | 99.8 | No change |

Conclusion: The long-term accelerated experiments show that: standing at 25 °C with 60% RH and at 40 °C with 75% RH for 6 months, the free-state crystal form A exhibited both good physical stability and good chemical stability.

### Experimental Example 3. Influencing Factor Experiments of Crystal Form B of Compound

The crystal form B of (S)-6-((1-(2-fluoro-5-methylphenyl)ethyl)amino)-3-(tetrahydro-2H-pyran-4-yl)-1,3,5-triazine-2,4(1H,3H)-dione was spread out and left to stand in open flasks, and the samples were subjected to 30-day stability tests under high temperature (40 °C and 60 °C), light exposure, and high humidity (RH 75% and RH 90%) conditions.

**Table 11. The influencing factor stability of the crystal form B of the compound**

| Conditions | Time (days) | Color and state | Purity% | Crystal form |
|---|---|---|---|---|
| Initial | 0 | Off-white solid | 99.9 | Crystal form B |
| Light exposure (4500 Lux) | 5 | Off-white solid | 99.9 | No change |
| | 10 | Off-white solid | 99.9 | No change |
| | 30 | Off-white solid | 99.9 | No change |
| 40 °C | 5 | Off-white solid | 99.8 | No change |
| | 10 | Off-white solid | 99.9 | No change |
| | 30 | Off-white solid | 99.9 | No change |
| 60 °C | 5 | Off-white solid | 99.9 | No change |
| | 10 | Off-white solid | 99.9 | No change |
| | 30 | Off-white solid | 99.9 | No change |
| 75% RH | 5 | Off-white solid | 99.8 | No change |
| | 10 | Off-white solid | 99.8 | No change |
| | 30 | Off-white solid | 99.8 | No change |
| 92.5% RH | 5 | Off-white solid | 99.9 | No change |
| | 10 | Off-white solid | 99.9 | No change |
| | 30 | Off-white solid | 99.9 | No change |

Conclusion: The influencing factor experiments show that: standing under light exposure, high temperature (40 °C and 60 °C), and high humidity (75% RH and 92.5% RH) conditions for 30 days, the free-state crystal form B exhibited both good physical stability and good chemical stability.

### Experimental Example 4. Long-Term/Accelerated Stability Experiments of Crystal Form B of Compound

The crystal form B of (S)-6-((1-(2-fluoro-5-methylphenyl)ethyl)amino)-3-(tetrahydro-2H-pyran-4-yl)-1,3,5-triazine-2,4(1H,3H)-dione was subjected to 6-month long-term accelerated stability tests under long-term (25 °C, 60% RH) and accelerated (40 °C, 75% RH) conditions.

**Table 12. The results of the long-term accelerated stability experiments of the crystal form B of the compound**

| Test conditions | Time (months) | Purity (%) | Crystal form |
|---|---|---|---|
| Initial | | 99.9 | Crystal form B |
| 25 °C | 1 | 99.8 | No change |
| | 2 | 99.8 | No change |
| 60% RH | 3 | 99.9 | No change |
| | 6 | 99.9 | No change |
| 40 °C | 1 | 99.9 | No change |
| | 2 | 99.9 | No change |
| 75% RH | 3 | 99.9 | No change |
| | 6 | 99.9 | No change |

Conclusion: The long-term accelerated experiments show that: standing at 25 °C with 60% RH and at 40 °C with 75% RH for 6 months, the free-state crystal form B exhibited both good physical stability and good chemical stability.

## Claims

1. A crystal form A of (S)-6-((1-(2-fluoro-5-methylphenyl)ethyl)amino)-3-(tetrahydro-2H-pyran-4-yl)-1,3,5-triazine-2,4(1H,3H)-dione, wherein an X-ray powder diffraction pattern represented by 2θ angles, which are diffraction angles, of the crystal form has characteristic peaks at 9.2, 10.9, 18.6, 20.1, 22.0, and 26.9, preferably 9.2, 10.9, 13.3, 16.9, 18.6, 19.2, 20.1, 22.0, 26.9, and 35.6, and more preferably 9.2, 10.9, 13.3, 16.9, 18.6, 19.2, 20.1, 20.6, 22.0, 26.9, 28.0, and 35.6; most preferably, the X-ray powder diffraction pattern represented by 2θ angles, which are diffraction angles, is shown in FIG. 2.

2. A crystal form B of (S)-6-((1-(2-fluoro-5-methylphenyl)ethyl)amino)-3-(tetrahydro-2H-pyran-4-yl)-1,3,5-triazine-2,4(1H,3H)-dione, wherein an X-ray powder diffraction pattern represented by 2θ angles, which are diffraction angles, of the crystal form has characteristic peaks at 11.1, 15.9, 18.8, 20.4, 22.2, and 26.8, preferably 6.6, 9.5, 11.1, 15.9, 18.8, 20.4, 21.0, 22.2, 24.6, and 26.8, and more preferably 6.6, 9.5, 11.1, 13.3, 14.0, 15.9, 18.8, 19.0, 19.8, 20.4, 21.0, 22.2, 26.8, and 28.0; most preferably, the X-ray powder diffraction pattern represented by 2θ angles, which are diffraction angles, is shown in FIG. 3.

3. A crystal form C of (S)-6-((1-(2-fluoro-5-methylphenyl)ethyl)amino)-3-(tetrahydro-2H-pyran-4-yl)-1,3,5-triazine-2,4(1H,3H)-dione, wherein an X-ray powder diffraction pattern represented by 2θ angles, which are diffraction angles, of the crystal form has characteristic peaks at 8.3, 14.4, 16.8, 17.8, 19.3, 22.3, and 23.1; most preferably, the X-ray powder diffraction pattern represented by 2θ angles, which are diffraction angles, is shown in FIG. 4.

4. A crystal form D of (S)-6-((1-(2-fluoro-5-methylphenyl)ethyl)amino)-3-(tetrahydro-2H-pyran-4-yl)-1,3,5-triazine-2,4(1H,3H)-dione, wherein an X-ray powder diffraction pattern represented by 2θ angles, which are diffraction angles, of the crystal form has characteristic peaks at 7.8, 8.7, 9.7, 13.5, 14.0, and 22.9, preferably 7.8, 8.7, 9.7, 13.5, 14.0, 16.7, 19.6, 20.2, and 22.9, and more preferably 7.8, 8.7, 9.7, 13.5, 14.0, 15.5, 16.7, 17.5, 19.6, 20.2, 22.9, and 23.9; most preferably, the X-ray powder diffraction pattern represented by 2θ angles, which are diffraction angles, is shown in FIG. 5.

5. A crystal form E of (S)-6-((1-(2-fluoro-5-methylphenyl)ethyl)amino)-3-(tetrahydro-2H-pyran-4-yl)-1,3,5-triazine-2,4(1H,3H)-dione, wherein an X-ray powder diffraction pattern represented by 2θ angles, which are diffraction angles, of the crystal form has characteristic peaks at 6.9, 9.3, 14.2, 17.1, 18.0, and 25.0, preferably 6.9, 9.3, 13.5, 14.2, 17.1, 18.0, 22.4, 23.2, and 25.0, and more preferably 6.9, 9.3, 13.5, 14.2, 16.1, 17.1, 18.0, 21.1, 22.4, 23.2, 23.8, and 25.0; most preferably, the X-ray powder diffraction pattern represented by 2θ angles, which are diffraction angles, is shown in FIG. 6.

6. The crystal form of (S)-6-((1-(2-fluoro-5-methylphenyl)ethyl)amino)-3-(tetrahydro-2H-pyran-4-yl)-1,3,5-triazine-2,4(1H,3H)-dione according to any one of claims 1-5, wherein the 2θ angles have a margin of error of ±0.2.

7. A preparation method for preparing the crystal form A, B, C, D, or E according to any one of claims 1-6, wherein the method is selected from the group consisting of any one of the following methods:
method 1:
(a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent, and dissolving the compound by stirring or heat, and
(b) crystallizing;
or, method 2:
(a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent, and dissolving the compound by stirring or heat, and
(b) adding a second solvent, and crystallizing;
or, method 3:
(a) mixing the compound (S)-6-((1-(2-fluoro-5-methylphenyl)ethyl)amino)-3-(tetrahydro-2H-pyran-4-yl)-1,3,5-triazine-2,4(1H,3H)-dione with a solvent, and
(b) stirring and triturating, and crystallizing.

8. A pharmaceutical composition, comprising the following components:
i) the crystal form of (S)-6-((1-(2-fluoro-5-methylphenyl)ethyl)amino)-3-(tetrahydro-2H-pyran-4-yl)-1,3,5-triazine-2,4(1H,3H)-dione according to any one of claims 1-6; and
ii) one or more pharmaceutically acceptable excipients.

9. A method for preparing a pharmaceutical composition, comprising the following step: a step of mixing the crystal form of (S)-6-((1-(2-fluoro-5-methylphenyl)ethyl)amino)-3-(tetrahydro-2H-pyran-4-yl)-1,3,5-triazine-2,4(1H,3H)-dione according to any one of claims 1-6 with a pharmaceutically acceptable excipient.

10. Use of the crystal forms of (S)-6-((1-(2-fluoro-5-methylphenyl)ethyl)amino)-3-(tetrahydro-2H-pyran-4-yl)-1,3,5-triazine-2,4(1H,3H)-dione according to claims 1-6 or the composition according to claim 8 in the preparation of a medicament for treating a disease or disorder associated with myosin regulation.

11. Use of the crystal forms of (S)-6-((1-(2-fluoro-5-methylphenyl)ethyl)amino)-3-(tetrahydro-2H-pyran-4-yl)-1,3,5-triazine-2,4(1H,3H)-dione according to claims 1-6 or the composition according to claim 8 in the preparation of a medicament for treating a disease or disorder, wherein the disease or disorder is selected from the group consisting of diastolic heart failure with preserved ejection fraction, ischemic heart disease, angina pectoris, restrictive cardiomyopathy, diastolic dysfunction, hypertrophic cardiomyopathy (HCM), non-obstructive hypertrophic cardiomyopathy (nHCM), obstructive hypertrophic cardiomyopathy (oHCM), valvular diseases, heart failure with preserved ejection fraction (HFpEF), heart failure with mid-range ejection fraction (HFmREF), aortic stenosis, inflammatory cardiomyopathy, Löeffler endocarditis, endomyocardial fibrosis, invasive cardiomyopathy, hemochromatosis, Fabry disease, glycogen storage disease, congenital heart defect, tetralogy of Fallot, left ventricular hypertrophy, refractory angina, and Chagas disease, optionally from the group consisting of ischemic heart disease, restrictive cardiomyopathy, hypertrophic cardiomyopathy (HCM), non-obstructive hypertrophic cardiomyopathy (nHCM), obstructive hypertrophic cardiomyopathy (oHCM), inflammatory cardiomyopathy, invasive cardiomyopathy, congenital heart defect, and left ventricular hypertrophy; optionally, the disease or disorder is hypertrophic cardiomyopathy (HCM).
